(19) Europäisches Patentamt
European Patent Office
Office européen des brevets

(11) EP 4 148 738 A1

(12) EUROPEAN PATENT APPLICATION

(43) Date of publication:
15.03.2023 Bulletin 2023/11

(51) International Patent Classification (IPC):
G16B 15/20 (2019.01)

(21) Application number: 22176981.3

(52) Cooperative Patent Classification (CPC):
G16B 15/20

(22) Date of filing: 02.06.2022

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 13.09.2021 JP 2021148849

(71) Applicant: FUJITSU LIMITED
Kawasaki-shi, Kanagawa 211-8588 (JP)

(72) Inventors:
• Manabe, Toshio
Kawasaki-shi, Kanagawa, 211-8588 (JP)
• Sato, Hiroyuki
Kawasaki-shi, Kanagawa, 211-8588 (JP)

(74) Representative: Hoffmann Eitle
Patent- und Rechtsanwälte PartmbB
Arabellastraße 30
81925 München (DE)

(54) SEARCH PROGRAM, SEARCH METHOD, AND SEARCH DEVICE

(57) A search program that causes at least one computer to execute a process, the process includes searching for, as an answer of a combinatorial optimization problem, a first arrangement of a plurality of amino acids included in a medium molecule based on a value of a first cost arithmetic expression that does not make distinction between a L-form and a D-form of the plurality of amino acids; searching for, as the answer of the combinatorial optimization problem, a second arrangement of the plurality of amino acids based on a value of a second cost arithmetic expression that makes distinction between the L-form and the D-form of the plurality of amino acids by setting the first arrangement as an initial arrangement of searching; and outputting the second arrangement.

FIG. 1

**Description**

FIELD

The embodiments discussed herein are related to a search program, a search method, and a search device.

BACKGROUND

In recent years, in the field of drug discovery, drug discovery using medium molecules (molecular weight 500 to 3000) with few side effects is expected, and the development of a search method for searching for a stable structure of medium molecules is underway.

As an example, there is a search method that applies an interaction potential of a plurality of amino acids to a coarse-grained model of a medium molecule containing a plurality of amino acids and solves the optimum arrangement of amino acids that minimizes the value of a cost arithmetic expression in a lattice space divided in a lattice shape, as a combinatorial optimization problem. According to the search method, an optimum arrangement combination in the coarse-grained model may be efficiently found by a search.

Japanese Laid-open Patent Publication No. 2019-159683, Japanese Laid-open Patent Publication No. 2015-007537, and Japanese Laid-open Patent Publication No. 2021-082165 are disclosed as related art.

SUMMARY

[TECHNICAL PROBLEM]

Meanwhile, even in a case where the above search method is used, for example, when trying to search for an optimum arrangement combination of amino acids in a coarse-grained model such as a peptide having a large number of residues, the search time increases because the number of bits used for the combinatorial optimization problem becomes enormous.

In one aspect, the aim is to efficiently find an optimum arrangement combination in a coarse-grained model of a medium molecule by a search.

[SOLUTION TO PROBLEM]

According to an aspect of the embodiments, a search program that causes at least one computer to execute a process, the process includes searching for, as an answer of a combinatorial optimization problem, a first arrangement of a plurality of amino acids included in a medium molecule based on a value of a first cost arithmetic expression that does not make distinction between a L-form and a D-form of the plurality of amino acids; searching for, as the answer of the combinatorial optimization problem, a second arrangement of the plurality of amino acids based on a value of a second cost arithmetic expression that makes distinction between the L-form and the D-form of the plurality of amino acids by setting the first arrangement as an initial arrangement of searching; and outputting the second arrangement.

[ADVANTAGEOUS EFFECTS OF INVENTION]

An optimum arrangement combination in a coarse-grained model of a medium molecule may be efficiently found by a search.

BRIEF DESCRIPTION OF DRAWINGS

FIG. 1 is a diagram illustrating an example of the system configuration of a search system;
FIG. 2 is a diagram illustrating an example of the hardware configuration of a terminal device;
FIG. 3 is a diagram illustrating an example of cost arithmetic expression information;
FIG. 4 is a diagram illustrating an example of constraint term arithmetic expression information;
FIG. 5 is a diagram illustrating an example of the functional configuration of a terminal device;
FIG. 6 is a diagram illustrating an example of the functional configuration of an Ising device;
FIG. 7 is a flowchart illustrating a flow of a search process; and
FIGs. 8A and 8B are diagrams illustrating specific examples of the search process.

DESCRIPTION OF EMBODIMENTS

Hereinafter, each embodiment will be described with reference to the accompanying drawings. Note that, in this specification and the drawings, components having substantially the same functional configuration are denoted by the same reference signs, and redundant description will be omitted.

[First Embodiment]

<System Configuration of Search System>

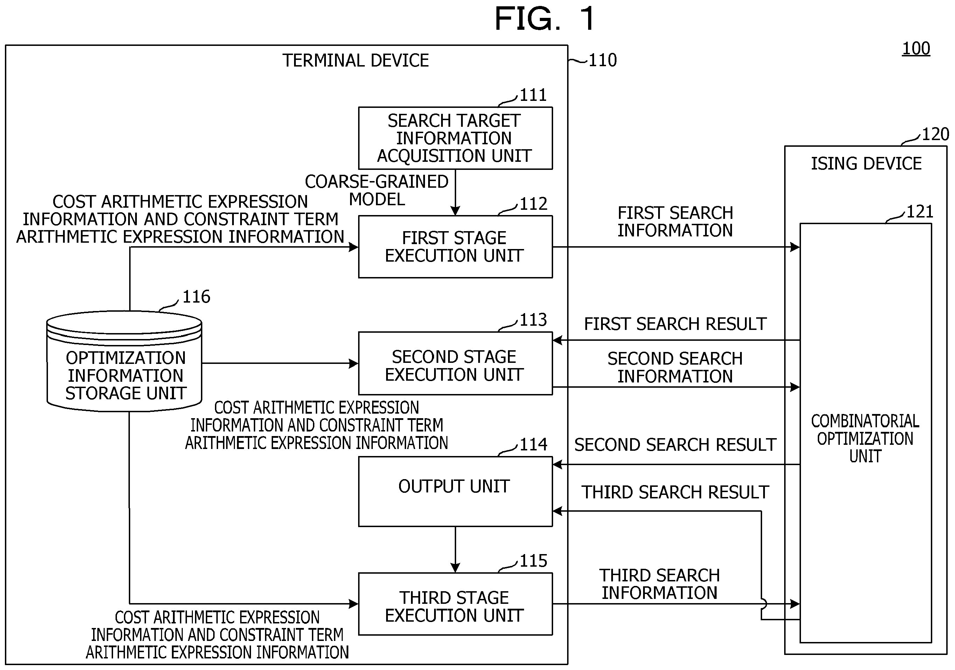

First, a system configuration of a search system according to a first embodiment will be described. FIG. 1 is a diagram illustrating an example of the system configuration of the search system. A search system 100 is a system that searches for an optimum arrangement combination that minimizes the cost under predetermined constraint conditions in a lattice space, using a coarse-grained model that coarse-grains the molecular structure of a medium molecule, as a search target.

As illustrated in FIG. 1, the search system 100 includes a terminal device 110 (an example of a search device) and an Ising device 120 such as a quantum annealing device.

A search program is installed in the terminal device 110, and when the program is executed, the terminal device 110 functions as a search target information acquisition unit 111, a first stage execution unit 112, a second stage execution unit 113, an output unit 114, and a third stage execution unit 115.

The search target information acquisition unit 111 acquires a search target coarse-grained model of a medium molecule for which a search for an optimum arrangement combination is to be made. In the first embodiment, the search target coarse-grained model of the medium molecule acquired by the search target information acquisition unit 111 is, for example, a model obtained by replacing each of ▪ a skeleton part centered on the $\alpha$-carbon of an amino acid forming the main chain particles of a peptide, and ▪ side chain particles with one coarse-grained particle, and ▪ arranging the coarse-grained particles in a space (lattice space) of a simple cubic lattice (face-centered cubic (FCC)). Note that bits denoting positions are allocated to each point in the lattice space, and once the arrangement of the coarse-grained particles is fixed, the distances between the coarse-grained particles are uniquely specified.

The search target information acquisition unit 111 notifies the first stage execution unit 112 of the search target acquired coarse-grained model of the medium molecule.

As information demanded by the Ising device 120 to solve the optimum arrangement combination of amino acids that minimizes the value of a cost arithmetic expression in the lattice space under predetermined constraint conditions, as a combinatorial optimization problem, the first stage execution unit 112 reads ▪ cost arithmetic expression information (details will be described later) and ▪ constraint term arithmetic expression information (details will be described later) from an optimization information storage unit 116.

In addition, for the search target coarse-grained model of the medium molecule, the first stage execution unit 112 instructs the Ising device 120 to search for the optimum arrangement combination from an initial arrangement specified in advance, using the cost arithmetic expression information and the constraint term arithmetic expression information. Upon instructing the Ising device 120, the first stage execution unit 112 transmits first search information to the Ising device 120. The first search information mentioned here includes a coarse-grained model in the initial arrangement specified in advance, the cost arithmetic expression information, and the constraint term arithmetic expression information.

Note that, in the first embodiment, the first stage execution unit 112 transforms the cost arithmetic expression information such that the computation amount (the number of bits) of the Ising device 120 is reduced when searching for an optimum arrangement combination and then transmits the transformed cost arithmetic expression information to the Ising device 120. For example, the cost arithmetic expression information contained in the first search information when the first stage execution unit 112 transmits the first search information is transformed cost arithmetic expression information that has been transformed so as to reduce the computation amount (the number of bits).

The second stage execution unit 113 acquires a first search result transmitted from the Ising device 120 in response to the first stage execution unit 112 instructing to search for an optimum arrangement combination.

In addition, the second stage execution unit 113 reads the cost arithmetic expression information and the constraint term arithmetic expression information from the optimization information storage unit 116. Furthermore, for the search target coarse-grained model of the medium molecule, the second stage execution unit 113 instructs the Ising device 120 to search for a further optimum arrangement combination, using the untransformed cost arithmetic expression information and the constraint term arithmetic expression information. Upon instructing the Ising device 120, the second stage execution unit 113 transmits second search information to the Ising device 120. The second search information mentioned here includes a coarse-grained model whose initial arrangement is the first search result, the untransformed cost arithmetic expression information, and the constraint term arithmetic expression information.

The output unit 114 acquires a second search result transmitted from the Ising device 120 in response to the second stage execution unit 113 instructing to search for an optimum arrangement combination and notifies the third stage execution unit 115 of the acquired second search result.

In addition, the output unit 114 evaluates the acquired second search result and determines whether or not there is room for improvement in the optimum arrangement combination. When it is determined that there is no room for improvement, the output unit 114 outputs the second search result as an optimum solution.

In addition, the output unit 114 acquires a third search result transmitted from the Ising device 120 in response to notifying the third stage execution unit 115 of the second search result and notifies the third stage execution unit 115 again of the acquired third search result.

Furthermore, the output unit 114 evaluates the acquired third search result and determines whether or not there is room for improvement in the optimum arrangement combination. When it is determined that there is no room for improvement, the output unit 114 outputs the third search result as an optimum solution.

The third stage execution unit 115 acquires the second search result or the third search result notified by the output unit 114.

In addition, the third stage execution unit 115 reads the cost arithmetic expression information and the constraint term arithmetic expression information from the optimization information storage unit 116. Furthermore, for the search target coarse-grained model of the medium molecule, the third stage execution unit 115 instructs the Ising device 120 to search for a further optimum arrangement combination, using the cost arithmetic expression information and the constraint term arithmetic expression information. Upon instructing the Ising device 120, the third stage execution unit 115 transmits third search information to the Ising device 120. The third search information mentioned here includes a coarse-grained model whose initial arrangement is the second search result (or the third search result), the cost arithmetic expression information, and the constraint term arithmetic expression information.

Note that, in the first embodiment, the third stage execution unit 115 changes a coefficient contained in the constraint term arithmetic expression information such that the Ising device 120 does not fall into a local solution when searching for an optimum arrangement combination and then transmits the changed constraint term arithmetic expression information to the Ising device 120. For example, the constraint term arithmetic expression information included when the third stage execution unit 115 transmits the third search information is changed constraint term arithmetic expression information in which the coefficient has been changed so as not to fall into a local solution.

Meanwhile, an optimization program is installed in the Ising device 120, and when the program is executed, the Ising device 120 functions as a combinatorial optimization unit 121.

When the first search information is received from the terminal device 110, the combinatorial optimization unit 121 calculates the first search result by solving the optimum arrangement combination in the coarse-grained model as a combinatorial optimization problem and transmits the calculated first search result to the terminal device 110. As described above, since the cost arithmetic expression information contained in the first search information is the transformed cost arithmetic expression information that has been transformed so as to reduce the computation amount (the number of bits), the combinatorial optimization unit 121 is allowed to calculate the first search result at high speed.

In addition, when the second search information is received from the terminal device 110, the combinatorial optimization unit 121 calculates the second search result by solving the optimum arrangement combination in the coarse-grained model as a combinatorial optimization problem and transmits the calculated second search result to the terminal device 110. As described above, since the coarse-grained model contained in the second search information has the first search result as the initial arrangement, the combinatorial optimization unit 121 is allowed to calculate the second search result at high speed. In addition, as described above, since the cost arithmetic expression information contained in the second search information is the untransformed cost arithmetic expression information, the combinatorial optimization unit 121 is allowed to calculate the second search result with high accuracy.

In addition, when the third search information is received from the terminal device 110, the combinatorial optimization unit 121 calculates the third search result by solving the optimum arrangement combination in the coarse-grained model as a combinatorial optimization problem and transmits the calculated third search result to the terminal device 110. As described above, since the constraint term arithmetic expression information contained in the third search information is the changed constraint term arithmetic expression information in which the coefficient has been changed, the combinatorial optimization unit 121 is allowed to calculate the third search result with high accuracy without falling into a local solution.

<Hardware Configuration of Terminal Device>

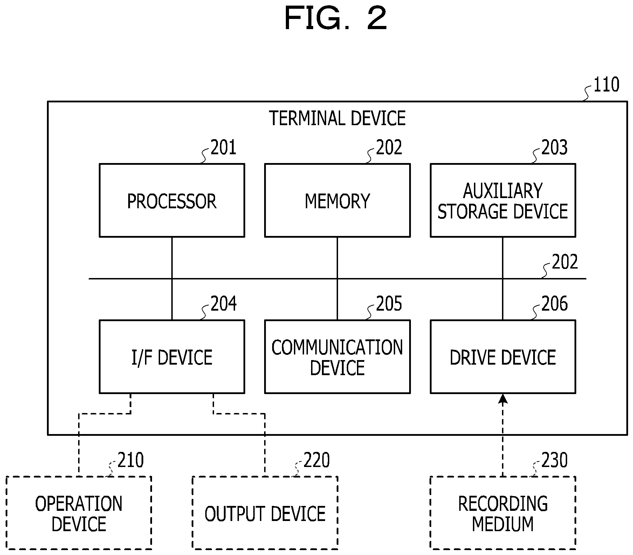

Next, a hardware configuration of the terminal device 110 will be described. FIG. 2 is a diagram illustrating an example of the hardware configuration of the terminal device.

As illustrated in FIG. 2, the terminal device 110 includes a processor 201, a memory 202, an auxiliary storage device 203, an interface (I/F) device 204, a communication device 205, and a drive device 206. Note that the respective pieces of hardware of the terminal device 110 are interconnected via a bus 207.

The processor 201 includes various arithmetic devices such as a central processing unit (CPU) and a graphics processing unit (GPU). The processor 201 reads various programs (for example, the search program and the like) into the memory 202 and executes the read programs.

The memory 202 includes a main storage device such as a read only memory (ROM) or a random access memory (RAM). The processor 201 and the memory 202 form a so-called computer. The processor 201 executes various programs read into the memory 202 to cause the computer to implement various functions.

The auxiliary storage device 203 stores various programs and various sorts of information used when the various programs are executed by the processor 201. For example, the optimization information storage unit 116 is implemented in the auxiliary storage device 203.

The I/F device 204 is a connection device that connects an operation device 210 and an output device 220, which are examples of external devices, with the terminal device 110.

The communication device 205 is a communication device for communicating with the Ising device 120, which is an example of another device.

The drive device 206 is a device for setting a recording medium 230. The recording medium 230 mentioned here includes a medium that optically, electrically, or magnetically records information, as in a compact disc read only memory (CD-ROM), a flexible disk, a magneto-optical disk, or the like. Alternatively, the recording medium 230 may include a semiconductor memory or the like that electrically records information, as in a ROM, a flash memory, or the like.

Note that various programs installed in the auxiliary storage device 203 are installed, for example, by setting the distributed recording medium 230 in the drive device 206 and reading the various programs recorded in the recording medium 230 by the drive device 206. Alternatively, the various programs to be installed in the auxiliary storage device 203 may be installed by being downloaded from a network via the communication device 205.

Note that only the hardware configuration of the terminal device 110 has been described here, and the hardware configuration of the Ising device 120 has not been described. However, the hardware configuration of the Ising device 120 may be similar to the hardware configuration of the terminal device 110, for example. In this case, the Ising device 120 functions as the combinatorial optimization unit 121 when the optimization program read into the memory is executed by the processor.

Alternatively, the hardware configuration of the Ising device 120 may be a hardware configuration similar to the hardware configuration of a so-called quantum computer.

<Specific Example of Cost Arithmetic Expression Information>

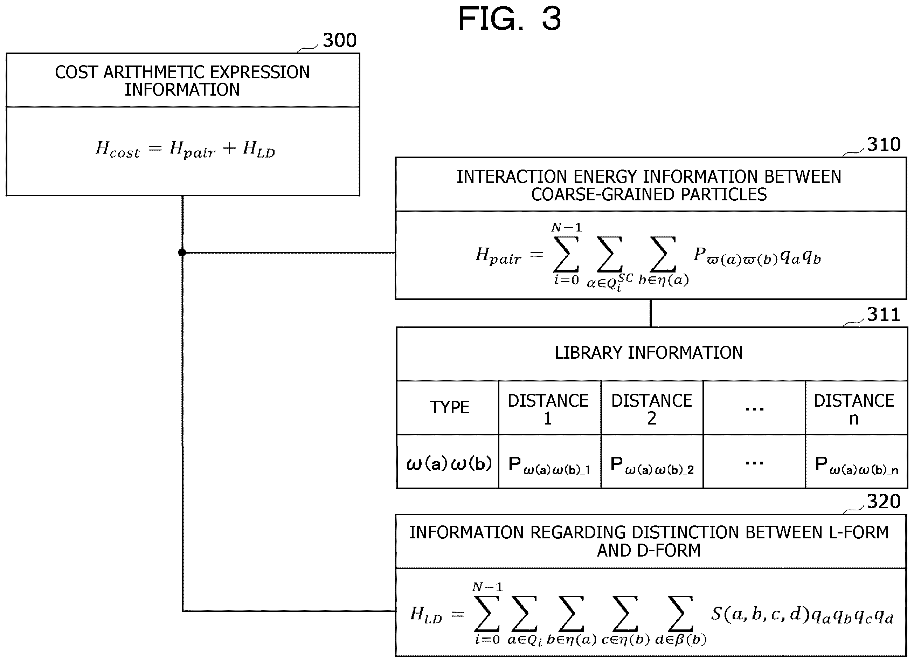

Next, a specific example of the cost arithmetic expression information stored in the optimization information storage unit 116 will be described. FIG. 3 is a diagram illustrating an example of the cost arithmetic expression information. As illustrated in cost arithmetic expression information 300 in FIG. 3, in the formulation for solving the optimum arrangement combination in the coarse-grained model as a combinatorial optimization problem by an annealing method, the cost is denoted by the Hamiltonian in following expression (1).

[Mathematical Expression 1]

$$H_{cost} = H_{pair} + H_{LD} \quad (1)$$

In this manner, the cost includes a term that represents the interaction energy between the coarse-grained particles and a term that represents the difference between the levorotatory (L)-form and the dextrorotatory (D)-form of the coarse-grained particles.

In addition, as illustrated in interaction energy information 310 between the coarse-grained particles in FIG. 3, the Hamiltonian in the term that represents the interaction energy between the coarse-grained particles is defined by following expression (2).

[Mathematical Expression 2]

$$H_{pair} = \sum_{i=0}^{N-1} \sum_{\alpha \in Q_i^{SC}} \sum_{b \in \eta(a)} P_{\varpi(a)\varpi(b)} q_a q_b \quad (2)$$

Note that, in above expression (2), . N: the total number of coarse-grained particles (amino acid residues), . $Q^i$: a set of bit numbers that represent the main chain particles of the i-th coarse-grained particle (amino acid residue),

- $Q^{SC}_i$: a set of bit numbers that represent the side chain particles of the i-th coarse-grained particle (amino acid residue), . $\eta(a)$: a set of bit numbers that represent the lattice points of the side chain particles within a distance from the lattice point represented by a bit number a where potential data exists, . $\omega(a)$: a coarse-grained particle (amino acid residue) represented by the bit number a, . $\omega(b)$: a coarse-grained particle (amino acid residue) represented by a bit number b, ▪ $P_{\omega(a)\omega(b)}$: interaction energy acting between the coarse-grained particle (amino acid residue) $\omega(a)$ and the coarse-grained particle (amino acid residue) $\omega(b)$, . $q_a$: a variable of the bit number a, and ▪ $q_b$: a variable of the bit number b are designated.

In addition, as illustrated in library information 311 in FIG. 3, the interaction energy $P_{\omega(a)\omega(b)}$ acting between the coarse-grained particle (amino acid residue) $\omega(a)$ and the coarse-grained particle (amino acid residue) $\omega(b)$ is computed in advance for each of the distances for each type of coarse-grained particles. The example of the library information 311 in FIG. 3 illustrates a state in which the interaction energy $P_{\omega(a)\omega(b)}$ between the coarse-grained particle $\omega(a)$ and the coarse-grained particle $\omega(b)$ is computed in advance, and distances 1 to n are organized as a library.

In addition, as illustrated in information 320 regarding the distinction between the L-form and the D-form in FIG. 3, the Hamiltonian in the term representing the difference between the L-form and the D-form of the coarse-grained particles (amino acid residues) is defined by following expression (3).

[Mathematical Expression 3]

$$H_{LD} = \sum_{i=0}^{N-1} \sum_{a\in Q_i} \sum_{b\in\eta(a)} \sum_{c\in\eta(b)} \sum_{d\in\beta(b)} S(a,b,c,d)q_a q_b q_c q_d \qquad (3)$$

Note that, in above expression (3), . $\eta(a)$: a bit that represents the lattice point of a main chain particle adjacent to the lattice point represented by the bit number a, ▪ $\eta(b)$: a bit that represents the lattice point of a main chain particle adjacent to the lattice point represented by the bit number b, ▪ $\beta(b)$: a bit that represents the lattice point of a side chain particle adjacent to the lattice point represented by the bit number b, ▪ S(a, b, c, d): the value of the potential, ▪ $q_c$: a variable of a bit number c, and ▪ $q_d$: a variable of a bit number d are designated.

<Specific Example of Constraint Term Arithmetic Expression Information>

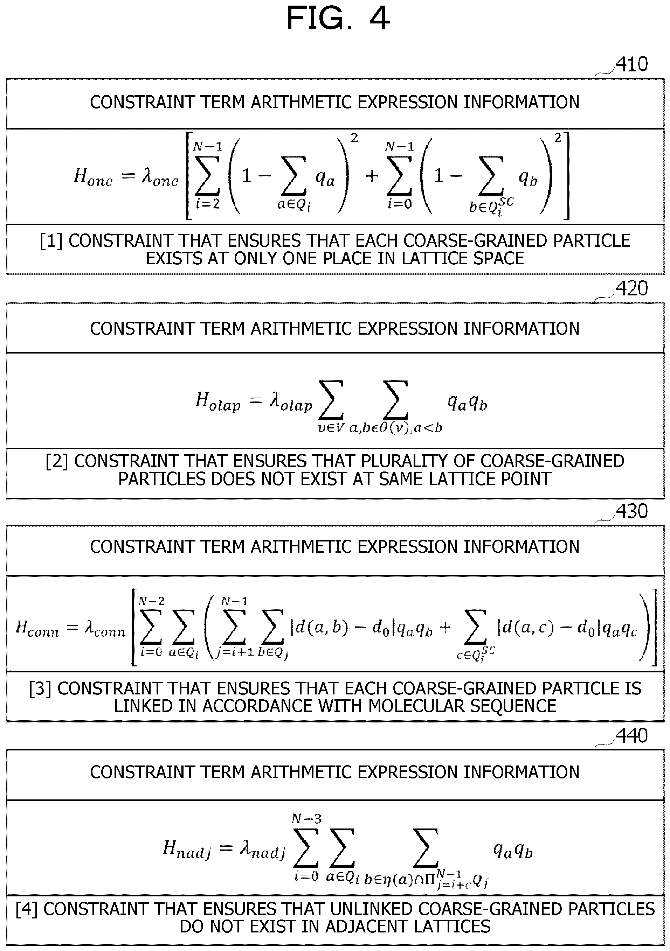

Next, a specific example of the constraint term arithmetic expression information stored in the optimization information storage unit 116 will be described. FIG. 4 is a diagram illustrating an example of the constraint term arithmetic expression information. As illustrated in FIG. 4, the optimization information storage unit 116 stores four types of constraint term arithmetic expression information.

Among these, constraint term arithmetic expression information 410 is a constraint condition that ensures that each coarse-grained particle (amino acid residue) exists at only one place in the lattice space and is defined by following expression (4).

[Mathematical Expression 4]

$$H_{one} = \lambda_{one} \left[ \sum_{i=2}^{N-1} \left(1 - \sum_{a\in Q_i} q_a\right)^2 + \sum_{i=0}^{N-1} \left(1 - \sum_{b\in Q_i^{SC}} q_b\right)^2 \right] \qquad (4)$$

Note that, in above expression (4), ▪ N: the total number of coarse-grained particles (amino acid residues), ▪ $Q^i$: a set of bit numbers that represent the main chain particles of the i-th coarse-grained particle (amino acid residue), ▪ $Q^{SC}_i$: a set of bit numbers that represent the side chain particles of the i-th coarse-grained particle (amino acid residue), ▪ $q_a$: a variable of the bit number a, ▪ $q_b$: a variable of the bit number b, and ▪ $\lambda_{one}$: a coefficient are designated.

In addition, constraint term arithmetic expression information 420 is a constraint condition that ensures that a plurality of coarse-grained particles (amino acid residues) does not exist at the same lattice point and is defined by following expression (5).

[Mathematical Expression 5]

$$H_{olap} = \lambda_{olap} \sum_{v \in V} \sum_{a,b \in \theta(v), a<b} q_a q_b \qquad (5)$$

Note that, in above expression (5), ▪ V: the lattice space, ▪ $\theta(v)$: a set of all bit numbers that represent the lattice points v, and ▪ $\lambda_{olap}$: a coefficient are designated.

In addition, constraint term arithmetic expression information 430 is a constraint condition that ensures that each coarse-grained particle (amino acid residue) is linked in accordance with the molecular sequence and is defined by following expression (6).

[Mathematical Expression 6]

$$H_{conn} = \lambda_{conn} \left[ \sum_{i=0}^{N-2} \sum_{a \in Q_i} \left( \sum_{j=i+1}^{N-1} \sum_{b \in Q_j} |d(a,b) - d_0| q_a q_b + \sum_{c \in Q_i^{SC}} |d(a,c) - d_0| q_a q_c \right) \right] (6)$$

Note that, in above expression (6), ▪ d(a, b): the distance between the lattice points corresponding to a-th and b-th bit variables, ▪ d(a, c): the distance between the lattice points corresponding to the a-th and c-th bit variables, ▪ do: the distance between nearest lattice points, ▪ $q_c$: a variable of the bit number c, ▪ $q_i$: a variable of a bit number i, ▪ $q_j$: a variable of a bit number j, and ▪ $\lambda_{conn}$: a coefficient are designated.

In addition, constraint term arithmetic expression information 440 is a constraint condition that ensures that unlinked coarse-grained particles (amino acid residues) do not exist in adjacent lattices and is defined by following expression (7).

[Mathematical Expression 7]

$$H_{nadj} = \lambda_{nadj} \sum_{i=0}^{N-3} \sum_{a \in Q_i} \sum_{b \in \eta(a) \cap \Pi_{j=i+c}^{N-1} Q_j} q_a q_b \qquad (7)$$

Note that, in above expression (7), ▪ $\eta(a)$: a set of bit numbers that represent adjacent lattice points of the lattice point represented by the a-th bit, ▪ c: 1 when an annular shape is formed and i = N/2 holds, and 2 in other cases, and ▪ $\lambda_{nadj}$: a coefficient are designated.

<Details of Functional Configuration of Terminal Device>

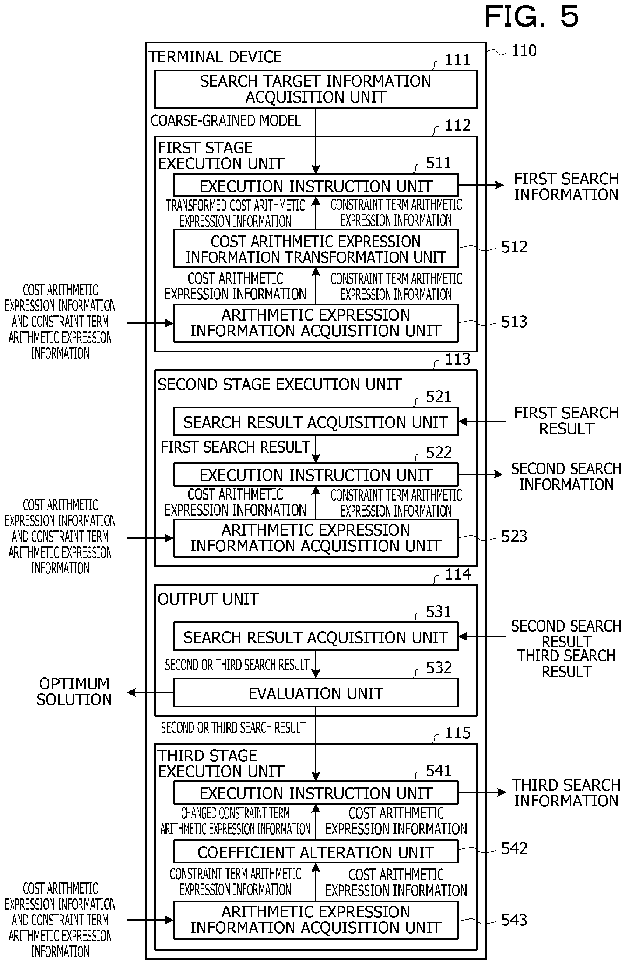

Next, the details of the functional configuration of the terminal device 110 will be described. FIG. 5 is a diagram illustrating an example of the functional configuration of the terminal device. As described with reference to FIG. 1, the terminal device 110 functions as the search target information acquisition unit 111, the first stage execution unit 112, the second stage execution unit 113, the output unit 114, and the third stage execution unit 115.

In addition, as illustrated in FIG. 5, the first stage execution unit 112 further includes an execution instruction unit 511, a cost arithmetic expression information transformation unit 512, and an arithmetic expression information acquisition unit 513.

In addition, the second stage execution unit 113 includes a search result acquisition unit 521, an execution instruction unit 522, and an arithmetic expression information acquisition unit 523.

In addition, the output unit 114 includes a search result acquisition unit 531 and an evaluation unit 532. Furthermore, the third stage execution unit 115 includes an execution instruction unit 541, a coefficient alteration unit 542, and an arithmetic expression information acquisition unit 543. Hereinafter, the details of each unit included in each of the first stage execution unit 112, the second stage execution unit 113, the output unit 114, and the third stage execution unit 115 will be described.

The arithmetic expression information acquisition unit 513 reads the cost arithmetic expression information and the constraint term arithmetic expression information from the optimization information storage unit 116 and notifies the cost arithmetic expression information transformation unit 512 of the read cost arithmetic expression information and constraint term arithmetic expression information.

The cost arithmetic expression information transformation unit 512 acquires the cost arithmetic expression information from the arithmetic expression information acquisition unit 513 and transforms the acquired cost arithmetic expression information so as to reduce the computation amount (the number of bits) of the Ising device 120 when searching for an optimum arrangement combination. For example, by omitting the term representing the difference between the L-form and the D-form of the coarse-grained particles in the cost arithmetic expression information 300, the cost arithmetic expression information transformation unit 512 generates the transformed cost arithmetic expression information indicated by following expression (8).
[Mathematical Expression 8]

$$H_{cost} = H_{pair} \qquad (8)$$

In the case of above expression (8), since the computation amount (the number of bits) can be reduced as compared with the cost arithmetic expression information indicated in above expression (1), the search time when the first search result is calculated at high speed may be significantly shortened.

In addition, the cost arithmetic expression information transformation unit 512 notifies the execution instruction unit 511 of the transformed cost arithmetic expression information and the constraint term arithmetic expression information.

The execution instruction unit 511 acquires a coarse-grained model in the initial arrangement specified in advance, from the search target information acquisition unit 111 and also acquires the transformed cost arithmetic expression information and the constraint term arithmetic expression information from the cost arithmetic expression information transformation unit 512. In addition, the execution instruction unit 511 transmits the first search information containing the coarse-grained model in the initial arrangement specified in advance, the transformed cost arithmetic expression information, and the constraint term arithmetic expression information, to the Ising device 120 and instructs the Ising device 120 to search for an optimum arrangement combination.

The search result acquisition unit 521 acquires the first search result from the Ising device 120 and notifies the execution instruction unit 522 of the acquired first search result.

The arithmetic expression information acquisition unit 523 reads the cost arithmetic expression information and the constraint term arithmetic expression information from the optimization information storage unit 116 and notifies the execution instruction unit 522 of the read cost arithmetic expression information and constraint term arithmetic expression information.

The execution instruction unit 522 acquires the first search result from the search result acquisition unit 521 and acquires the cost arithmetic expression information and the constraint term arithmetic expression information from the arithmetic expression information acquisition unit 523. In addition, the execution instruction unit 522 transmits the second search information containing a coarse-grained model whose initial arrangement is the acquired first search result, the untransformed cost arithmetic expression information, and the constraint term arithmetic expression information, to the Ising device 120 and instructs the Ising device 120 to search for an optimum arrangement combination. In the case of the second search information, a search for an optimum arrangement combination is made with the first search result as the initial arrangement. Therefore, even if the term representing the difference between the L-form and the D-form of the coarse-grained particles is included (for example, even for the arrangement considering the L-form and the D-form), the highly accurate second search result may be calculated at high speed.

The search result acquisition unit 531 acquires the second search result or the third search result from the Ising device 120 and notifies the evaluation unit 532 of the acquired second search result or third search result.

The evaluation unit 532 evaluates the second search result or the third search result notified by the search result acquisition unit 531 and determines whether or not there is room for improvement in the optimum arrangement combination. When it is determined that there is no room for improvement, the evaluation unit 532 outputs the immediately preceding search result as the optimum solution.

Note that, for the immediately preceding search result, for example, the second search result is output as the optimum solution in a case where a notification of the third search result is made after a notification of the second search result is made and the optimum arrangement combination has not been updated between the second search result and the third search result. In addition, for example, in a case where a notification of the k+1-th (k is any integer) third search result is made after a notification of the k-th third search result is made and the optimum arrangement combination has not been updated between the k-th time and the k+1-th time, the k-th third search result is output as the optimum solution.

Furthermore, when it is determined that there is room for improvement in the optimum arrangement combination, the evaluation unit 532 notifies the execution instruction unit 541 of the second search result or the third search result notified by the search result acquisition unit 531.

The arithmetic expression information acquisition unit 543 reads the cost arithmetic expression information and the constraint term arithmetic expression information from the optimization information storage unit 116 and notifies the coefficient alteration unit 542 of the read cost arithmetic expression information and constraint term arithmetic expression information.

The coefficient alteration unit 542 acquires the cost arithmetic expression information and the constraint term arithmetic expression information from the arithmetic expression information acquisition unit 543 and changes the coefficient contained in the constraint term arithmetic expression information. In addition, the coefficient alteration unit 542 notifies the execution instruction unit 541 of the changed constraint term arithmetic expression information and the cost arithmetic expression information.

The execution instruction unit 541 acquires the second search result or the third search result from the evaluation unit 532 and acquires the changed constraint term arithmetic expression information and the cost arithmetic expression information from the coefficient alteration unit 542. In addition, the execution instruction unit 541 transmits the third search information containing a coarse-grained model whose initial arrangement is the acquired second search result or third search result, the cost arithmetic expression information, and the changed constraint term arithmetic expression information, to the Ising device 120 and instructs the Ising device 120 to search for an optimum arrangement combination. In the case of the third search information, since a search for an optimum arrangement combination is made using the changed constraint term arithmetic expression information and the cost arithmetic expression information, a highly accurate search result may be calculated without falling into a local solution.

This is because the coarse-grained particles have positions and shapes different from the positions and shapes of actual molecules when arranged in an artificial lattice space, and accordingly, while there are cases where the local energy barrier is increased and it becomes difficult to shift to the vicinity of the optimum solution depending on the contribution of the constraint conditions, according to the third search information, it becomes easier to shift to the vicinity of the optimum solution, and the possibility of reaching a more accurate solution may be enhanced by ▪ the coefficient alteration unit 542 changing the coefficient of the constraint term arithmetic expression information, and ▪ temporarily weakening the constraint conditions.

<Details of Functional Configuration of Ising Device>

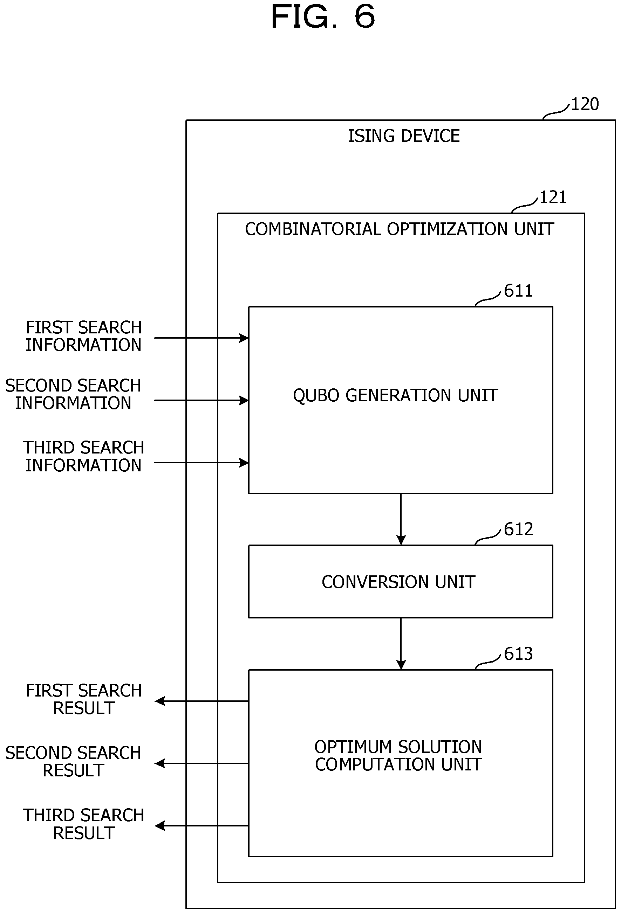

Next, the details of the functional configuration of the Ising device 120 will be described. FIG. 6 is a diagram illustrating an example of the functional configuration of the Ising device. As described with reference to FIG. 1, the Ising device 120 functions as the combinatorial optimization unit 121. In addition, as illustrated in FIG. 6, the combinatorial optimization unit 121 further includes a quadratic unconstrained binary optimization (QUBO) generation unit 611, a conversion unit 612, and an optimum solution computation unit 613.

The QUBO generation unit 611 acquires the first search information, the second search information, and the third search information from the terminal device 110. In addition, the QUBO generation unit 611 converts the transformed cost arithmetic expression information and the constraint term arithmetic expression information contained in the first search information into quadratic unconstrained binary optimization (QUBO). In addition, the QUBO generation unit 611 converts the untransformed cost arithmetic expression information and the constraint term arithmetic expression information contained in the second search information into QUBO. Furthermore, the QUBO generation unit 611 converts the cost arithmetic expression information and the changed constraint term arithmetic expression information contained in the third search information into QUBO.

The conversion unit 612 converts the converted QUBO into a format suitable for a digital annealer.

The optimum solution computation unit 613 searches for an optimum arrangement combination by solving the QUBO converted into a format suitable for the digital annealer, using the digital annealer. In addition, the optimum solution computation unit 613 transmits the optimum arrangement combination found by the search based on the first search information, to the terminal device 110 as the first search result. In addition, the optimum solution computation unit 613 transmits the optimum arrangement combination found by the search based on the second search information, to the terminal device 110 as the second search result. Furthermore, the optimum solution computation unit 613 transmits the optimum arrangement combination found by the search based on the third search information, to the terminal device 110 as the third search result.

<Flow of Search Process>

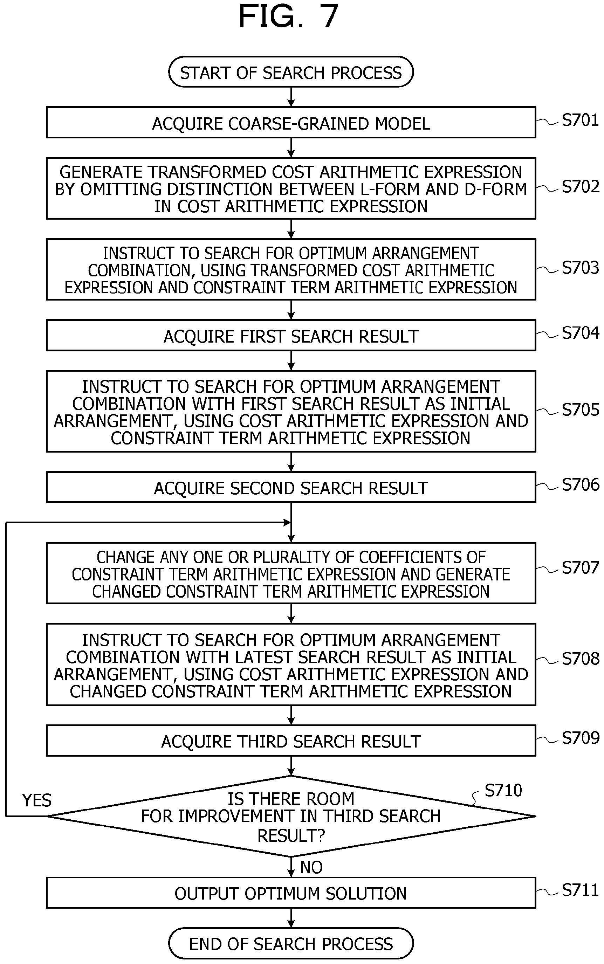

Next, a flow of the search process by the search system 100 will be described. FIG. 7 is a flowchart illustrating a flow of the search process.

In step S701, the search target information acquisition unit 111 of the terminal device 110 acquires a search target coarse-grained model of a medium molecule.

In step S702, the first stage execution unit 112 of the terminal device 110 generates the transformed cost arithmetic expression information in which the term representing the difference between the L-form and the D-form of the coarse-grained particles is omitted in the read cost arithmetic expression information 300.

In step S703, the first stage execution unit 112 of the terminal device 110 instructs the Ising device 120 to search for an optimum arrangement combination for the search target coarse-grained model of the medium molecule. At this time, the first stage execution unit 112 of the terminal device 110 instructs to search for an optimum arrangement combination using the transformed cost arithmetic expression information and the constraint term arithmetic expression information 410 to 440.

In step S704, the combinatorial optimization unit 121 of the Ising device 120 calculates the first search result, and the second stage execution unit 113 of the terminal device 110 acquires the first search result from the combinatorial optimization unit 121 of the Ising device 120.

In step S705, the second stage execution unit 113 of the terminal device 110 instructs the Ising device 120 to search for an optimum arrangement combination with the first search result as the initial arrangement, for the search target coarse-grained model of the medium molecule. At this time, the second stage execution unit 113 of the terminal device 110 instructs to search for an optimum arrangement combination using the cost arithmetic expression information 300 and the constraint term arithmetic expression information 410 to 440.

In step S706, the combinatorial optimization unit 121 of the Ising device 120 calculates the second search result, and the output unit 114 of the terminal device 110 acquires the second search result from the combinatorial optimization unit 121 of the Ising device 120.

In step S707, the third stage execution unit 115 of the terminal device 110 changes any one or a plurality of coefficients of the read constraint term arithmetic expression information 410 to 440 and generates the changed constraint term arithmetic expression information.

In step S708, the third stage execution unit 115 of the terminal device 110 instructs the Ising device 120 to search for an optimum arrangement combination with the latest search result acquired by the output unit 114, as the initial arrangement, for the search target coarse-grained model of the medium molecule. At this time, the third stage execution unit 115 of the terminal device 110 instructs to search for an optimum arrangement combination using the cost arithmetic expression information 300 and the changed constraint term arithmetic expression information.

In step S709, the combinatorial optimization unit 121 of the Ising device 120 calculates the third search result, and the output unit 114 of the terminal device 110 acquires the third search result from the combinatorial optimization unit 121 of the Ising device 120.

In step S710, the output unit 114 of the terminal device 110 determines whether or not there is room for improvement in the third search result acquired in step S709. When it is determined in step S710 that there is room for improvement (in the case of YES in step S710), the process returns to step S707.

In this case, the third stage execution unit 115 of the terminal device 110 generates the second changed constraint term arithmetic expression information (step S707) and instructs to search for an optimum arrangement combination with the first third search result as the initial arrangement (step S708). This causes the output unit 114 of the terminal device 110 to acquire the second third search result (step S709).

After that, in the search system 100, the processes in steps S707 to S710 are repeated until the output unit 114 of the terminal device 110 determines, in step S710, that there is no room for improvement.

On the other hand, when it is determined in step S710 that there is no room for improvement (in the case of NO in step S710), the process proceeds to step S711.

In step S711, the output unit 114 of the terminal device 110 outputs the search result immediately before determining that there is no room for improvement, as the optimum solution.

[Specific Example of Search Process]

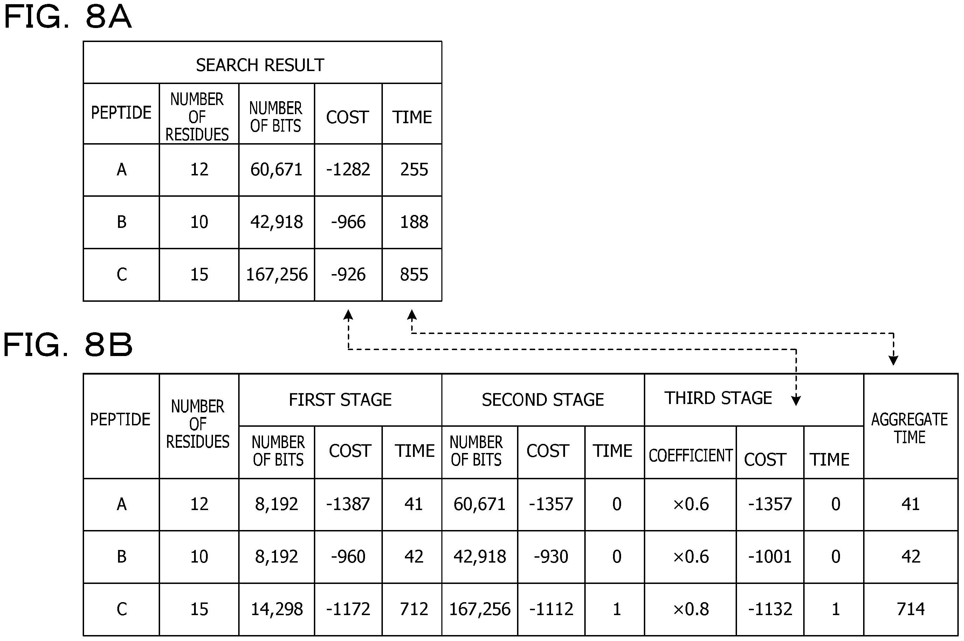

Next, a specific example of the search process by the search system 100 will be described. FIGs. 8A and 8B are diagrams illustrating specific examples of the search process and illustrate a state in which a search for an optimum arrangement combination was made with a coarse-grained model of three types of peptides (a peptide A (the number of residues = 12), a peptide B (the number of residues = 10), and a peptide C (the number of residues = 15)) as a search target.

Among these, FIG. 8A illustrates, as a comparative example, a case where a search for an optimum arrangement combination was made ▪ without using the transformed cost arithmetic expression information and ▪ without using the changed constraint term arithmetic expression information (which means illustrating a case where a search for an optimum arrangement combination was made using the cost arithmetic expression information 300 and the constraint term arithmetic expression information 410 to 440).

For example, it is indicated that, when the cost arithmetic expression information 300 and the constraint term arithmetic expression information 410 to 440 are used, the number of bits of the peptide A was "60,671", and by searching for an optimum arrangement combination, ▪ the cost was reduced by "1282", and ▪ the search time took "255" sec.

Similarly, it is indicated that, when the cost arithmetic expression information 300 and the constraint term arithmetic expression information 410 to 440 are used, the number of bits of the peptide B was "42,918", and by searching for an optimum arrangement combination, ▪ the cost was reduced by "966", and ▪ the search time took "188" sec.

Similarly, it is indicated that, when the cost arithmetic expression information 300 and the constraint term arithmetic expression information 410 to 440 are used, the number of bits of the peptide C was "167,256", and by searching for an optimum arrangement combination, ▪ the cost was reduced by "926", and ▪ the search time took "855" sec.

On the other hand, FIG. 8B illustrates a case where a search for an optimum arrangement combination was made by executing the search process illustrated in FIG. 7 (which means using the transformed cost arithmetic expression information and the changed constraint term arithmetic expression information).

For example, it is indicated that, when the transformed cost arithmetic expression information and the constraint term arithmetic expression information 410 to 440 are used in the first stage, the number of bits of the peptide A resulted in "8,192", and by searching for an optimum arrangement combination, ▪ the cost was reduced by "1387", and ▪ the search time took "41" sec.

Similarly, it is indicated that, when the transformed cost arithmetic expression information and the constraint term arithmetic expression information 410 to 440 are used in the first stage, the number of bits of the peptide B resulted in "8,192", and by searching for an optimum arrangement combination, ▪ the cost was reduced by "960", and ▪ the search time took "42" sec.

Similarly, it is indicated that, when the transformed cost arithmetic expression information and the constraint term arithmetic expression information 410 to 440 are used in the first stage, the number of bits of the peptide C resulted in "14,298", and by searching for an optimum arrangement combination, ▪ the cost was reduced by "1172", and ▪ the search time took "712" sec.

In addition, it is indicated that, when the untransformed cost arithmetic expression information 300 and the constraint term arithmetic expression information 410 to 440 are used in the second stage, the number of bits of the peptide A resulted in "60,671", but by searching for an optimum arrangement combination with the first search result as the initial arrangement, ▪ the cost was reduced by "1357", and ▪ the search time resulted in "0" sec.

Similarly, it is indicated that, when the cost arithmetic expression information 300 and the constraint term arithmetic expression information 410 to 440 are used in the second stage, the number of bits of the peptide B resulted in "42,918", but by searching for an optimum arrangement combination with the first search result as the initial arrangement, ▪ the cost was reduced by "930", and ▪ the search time resulted in "0" sec.

Similarly, it is indicated that, when the cost arithmetic expression information 300 and the constraint term arithmetic expression information 410 to 440 are used in the second stage, the number of bits of the peptide C resulted in "167,256", but by searching for an optimum arrangement combination with the first search result as the initial arrangement, ▪ the cost was reduced by "1112", and ▪ the search time resulted in "1" sec.

In addition, it is indicated that, when the cost arithmetic expression information 300 and the changed constraint term arithmetic expression information obtained by multiplying the coefficient by "0.6" are used in the third stage, by searching for an optimum arrangement combination with the second search result as the initial arrangement, ▪ the cost was reduced by "1357", and ▪ the search time resulted in "0" sec.

Similarly, it is indicated that, when the cost arithmetic expression information 300 and the changed constraint term arithmetic expression information obtained by multiplying the coefficient by "0.6" are used in the third stage, by searching for an optimum arrangement combination with the second search result as the initial arrangement, ▪ the cost was reduced by "1001", and ▪ the search time resulted in "0" sec.

Similarly, it is indicated that, when the cost arithmetic expression information 300 and the changed constraint term arithmetic expression information obtained by multiplying the coefficient by "0.8" are used in the third stage, by searching for an optimum arrangement combination with the second search result as the initial arrangement, ▪ the cost was reduced by "1132", and ▪ the search time resulted in "1" sec.

Contrasting FIGs. 8A and 8B, it was confirmed that ▪ the cost reduction amount is larger in FIG. 8B (refer to "cost" in "third stage" in FIG. 8B), and ▪ the search time is shorter in FIG. 8B (refer to "aggregate time" in FIG. 8B) for all of the peptides A to C. For example, according to the search system 100, an optimum arrangement combination in the coarse-grained model of the medium molecule may be found efficiently (at high speed and with high accuracy) by the search.

As is clear from the above description, the terminal device 110 according to the first embodiment instructs to search for an arrangement combination in the coarse-grained model of the medium molecule in multiple stages as a combinatorial optimization problem. At that time, in the first stage, the terminal device 110 according to the first embodiment instructs to search for an arrangement combination that minimizes the cost, by omitting the distinction between the L-form and the D-form of the amino acids to calculate the cost of the coarse-grained model. In addition, in the second stage, the terminal device 110 according to the first embodiment instructs to search for an arrangement that minimizes the cost, by calculating the cost of the coarse-grained model with the arrangement found by the search in the first stage as the initial arrangement, while making distinction between the L-form and the D-form of amino acids. Furthermore, the terminal device 110 according to the first embodiment outputs the arrangement found by the search in the second stage, as an optimum arrangement combination in the coarse-grained model.

Consequently, according to the first embodiment, a highly accurate arrangement combination may be found at high speed by the search. For example, according to the first embodiment, an optimum arrangement combination in the coarse-grained model of the medium molecule may be efficiently found by the search.

[Second Embodiment]

In the above first embodiment, the QUBO generation unit 611 has been described as sequentially converting the first search information and the second search information. However, the conversion timing by the QUBO generation unit 611 is not limited to this. For example, the transformed cost arithmetic expression information contained in the first search information and the untransformed cost arithmetic expression information contained in the second search information may be previously converted before the optimum solution computation unit 613 searches for an optimum arrangement combination.

In addition, in the above first embodiment, the search system 100 has been described as including the terminal device 110 and the Ising device 120. However, the search system 100 may be formed by a device in addition to the terminal device 110 and the Ising device 120 (which means three or more devices). Alternatively, the search system 100 may be formed by an integrated device (which means one device). In this case, the search process (FIG. 7) will be implemented, for example, by the one device executing the search program (the search program + the optimization program).

In addition, in the above first embodiment, the terminal device 110 has been described as including the search target information acquisition unit 111 to the third stage execution unit 115, and the Ising device 120 has been described as including the combinatorial optimization unit 121. However, some functions of the terminal device 110 may be implemented in the Ising device 120. Similarly, some functions of the Ising device 120 may be implemented in the terminal device 110.

Note that the embodiments are not limited to the configurations described here and may include, for example, combinations of the configurations or the like described in the above embodiments with other elements. These points may be altered without departing from the spirit of the embodiments and may be appropriately specified according to application modes thereof.

**Claims**

1. A search program that causes at least one computer to execute a process, the process comprising:

    searching for, as an answer of a combinatorial optimization problem, a first arrangement of a plurality of amino acids included in a medium molecule based on a value of a first cost arithmetic expression that does not make distinction between a L-form and a D-form of the plurality of amino acids;
    searching for, as the answer of the combinatorial optimization problem, a second arrangement of the plurality of amino acids based on a value of a second cost arithmetic expression that makes distinction between the L-form and the D-form of the plurality of amino acids by setting the first arrangement as an initial arrangement of searching; and
    outputting the second arrangement.

2. The search program according to claim 1, wherein the process further comprising:

    searching for a third arrangement of the plurality of amino acids by changing a coefficient of a certain constraint term among a plurality of constraint terms that constrains an arrangement of the plurality of amino acids; and
    outputting the third arrangement when the third arrangement is different from the second arrangement.

3. The search program according to claim 2, wherein
   the searching for the third arrangement includes searching for the third arrangement by lowering the coefficient of the certain constraint term.

4. The search program according to claim 2, wherein
   the plurality of constraint terms includes constraint terms that ensure that each of the plurality of amino acids included in the medium molecule exists at only one place in a lattice space.

5. The search program according to claim 2, wherein
   the plurality of constraint terms includes constraint terms that ensure that each of the plurality of amino acids included in the medium molecule does not exist at a same lattice point.

6. The search program according to claim 2, wherein
the plurality of constraint terms includes constraint terms that ensure that amino acids of the plurality of amino acids included in the medium molecule are interlinked in a certain sequence.

7. The search program according to claim 2, wherein
the plurality of constraint terms includes constraint terms that ensure that unlinked amino acids of the plurality of amino acids contained in the medium molecule do not exist in adjacent lattices.

8. The search program according to claim 3, wherein the outputting the third arrangement includes
when the third arrangement found by the search in which the coefficient of the constraint terms is lowered at a k+1-th time is same as the third arrangement found by the search in which the coefficient of the constraint terms is lowered at a k-th time, outputting the third arrangement found by the search in which the coefficient of the constraint terms is lowered at the k-th time, the k being any integer.

9. A search method for a computer to execute a process comprising:

searching for, as an answer of a combinatorial optimization problem, a first arrangement of a plurality of amino acids included in a medium molecule based on a value of a first cost arithmetic expression that does not make distinction between a L-form and a D-form of the plurality of amino acids;
searching for, as the answer of the combinatorial optimization problem, a second arrangement of the plurality of amino acids based on a value of a second cost arithmetic expression that makes distinction between the L-form and the D-form of the plurality of amino acids by setting the first arrangement as an initial arrangement of searching; and
outputting the second arrangement.

10. A search device comprising:

a first stage execution unit configured to search for, as an answer of a combinatorial optimization problem, a first arrangement of a plurality of amino acids included in a medium molecule based on a value of a first cost arithmetic expression that does not make distinction between a L-form and a D-form of the plurality of amino acids;
a second stage execution unit configured to search for, as the answer of the combinatorial optimization problem, a second arrangement of the plurality of amino acids based on a value of a second cost arithmetic expression that makes distinction between the L-form and the D-form of the plurality of amino acids by setting the first arrangement as an initial arrangement of searching; and
an output unit configured to output the second arrangement.

# FIG. 1

# FIG. 2

# FIG. 3

COST ARITHMETIC EXPRESSION INFORMATION `300`

$$H_{cost} = H_{pair} + H_{LD}$$

INTERACTION ENERGY INFORMATION BETWEEN COARSE-GRAINED PARTICLES `310`

$$H_{pair} = \sum_{i=0}^{N-1} \sum_{a \in Q_i^{SC}} \sum_{b \in \eta(a)} P_{\varpi(a)\varpi(b)} q_a q_b$$

LIBRARY INFORMATION `311`

| TYPE | DISTANCE 1 | DISTANCE 2 | ... | DISTANCE n |
|---|---|---|---|---|
| $\omega(a)\omega(b)$ | $P_{\omega(a)\omega(b)\_1}$ | $P_{\omega(a)\omega(b)\_2}$ | ... | $P_{\omega(a)\omega(b)\_n}$ |

INFORMATION REGARDING DISTINCTION BETWEEN L-FORM AND D-FORM `320`

$$H_{LD} = \sum_{i=0}^{N-1} \sum_{a \in Q_i} \sum_{b \in \eta(a)} \sum_{c \in \eta(b)} \sum_{d \in \beta(b)} S(a,b,c,d) q_a q_b q_c q_d$$

EP 4 148 738 A1

# FIG. 4

410

| CONSTRAINT TERM ARITHMETIC EXPRESSION INFORMATION |
|---|
| $$H_{one} = \lambda_{one} \left[ \sum_{i=2}^{N-1} \left( 1 - \sum_{a \in Q_i} q_a \right)^2 + \sum_{i=0}^{N-1} \left( 1 - \sum_{b \in Q_i^{SC}} q_b \right)^2 \right]$$ |
| [1] CONSTRAINT THAT ENSURES THAT EACH COARSE-GRAINED PARTICLE EXISTS AT ONLY ONE PLACE IN LATTICE SPACE |

420

| CONSTRAINT TERM ARITHMETIC EXPRESSION INFORMATION |
|---|
| $$H_{olap} = \lambda_{olap} \sum_{v \in V} \sum_{a,b \in \theta(v), a < b} q_a q_b$$ |
| [2] CONSTRAINT THAT ENSURES THAT PLURALITY OF COARSE-GRAINED PARTICLES DOES NOT EXIST AT SAME LATTICE POINT |

430

| CONSTRAINT TERM ARITHMETIC EXPRESSION INFORMATION |
|---|
| $$H_{conn} = \lambda_{conn} \left[ \sum_{i=0}^{N-2} \sum_{a \in Q_i} \left( \sum_{j=i+1}^{N-1} \sum_{b \in Q_j} |d(a,b) - d_0| q_a q_b + \sum_{c \in Q_i^{SC}} |d(a,c) - d_0| q_a q_c \right) \right]$$ |
| [3] CONSTRAINT THAT ENSURES THAT EACH COARSE-GRAINED PARTICLE IS LINKED IN ACCORDANCE WITH MOLECULAR SEQUENCE |

440

| CONSTRAINT TERM ARITHMETIC EXPRESSION INFORMATION |
|---|
| $$H_{nadj} = \lambda_{nadj} \sum_{i=0}^{N-3} \sum_{a \in Q_i} \sum_{b \in \eta(a) \cap \Pi_{j=i+c}^{N-1} Q_j} q_a q_b$$ |
| [4] CONSTRAINT THAT ENSURES THAT UNLINKED COARSE-GRAINED PARTICLES DO NOT EXIST IN ADJACENT LATTICES |

# FIG. 5

TERMINAL DEVICE — 111 ⌐ 110

SEARCH TARGET INFORMATION ACQUISITION UNIT

COARSE-GRAINED MODEL — 112

FIRST STAGE EXECUTION UNIT — 511

EXECUTION INSTRUCTION UNIT → FIRST SEARCH INFORMATION

TRANSFORMED COST ARITHMETIC EXPRESSION INFORMATION ↑ CONSTRAINT TERM ARITHMETIC EXPRESSION INFORMATION

COST ARITHMETIC EXPRESSION INFORMATION TRANSFORMATION UNIT — 512

COST ARITHMETIC EXPRESSION INFORMATION ↑ CONSTRAINT TERM ARITHMETIC EXPRESSION INFORMATION

COST ARITHMETIC EXPRESSION INFORMATION AND CONSTRAINT TERM ARITHMETIC EXPRESSION INFORMATION →

ARITHMETIC EXPRESSION INFORMATION ACQUISITION UNIT — 513

— 113

SECOND STAGE EXECUTION UNIT — 521

SEARCH RESULT ACQUISITION UNIT ← FIRST SEARCH RESULT

FIRST SEARCH RESULT — 522

EXECUTION INSTRUCTION UNIT → SECOND SEARCH INFORMATION

COST ARITHMETIC EXPRESSION INFORMATION ↑ CONSTRAINT TERM ARITHMETIC EXPRESSION INFORMATION

COST ARITHMETIC EXPRESSION INFORMATION AND CONSTRAINT TERM ARITHMETIC EXPRESSION INFORMATION →

ARITHMETIC EXPRESSION INFORMATION ACQUISITION UNIT — 523

— 114

OUTPUT UNIT — 531

SEARCH RESULT ACQUISITION UNIT ← SECOND SEARCH RESULT THIRD SEARCH RESULT

SECOND OR THIRD SEARCH RESULT — 532

OPTIMUM SOLUTION ← EVALUATION UNIT

SECOND OR THIRD SEARCH RESULT — 115

THIRD STAGE EXECUTION UNIT — 541

EXECUTION INSTRUCTION UNIT → THIRD SEARCH INFORMATION

CHANGED CONSTRAINT TERM ARITHMETIC EXPRESSION INFORMATION ↑ COST ARITHMETIC EXPRESSION INFORMATION

COEFFICIENT ALTERATION UNIT — 542

CONSTRAINT TERM ARITHMETIC EXPRESSION INFORMATION ↑ COST ARITHMETIC EXPRESSION INFORMATION

COST ARITHMETIC EXPRESSION INFORMATION AND CONSTRAINT TERM ARITHMETIC EXPRESSION INFORMATION →

ARITHMETIC EXPRESSION INFORMATION ACQUISITION UNIT — 543

# FIG. 6

ISING DEVICE — 120

COMBINATORIAL OPTIMIZATION UNIT — 121

FIRST SEARCH INFORMATION →

SECOND SEARCH INFORMATION →

THIRD SEARCH INFORMATION →

QUBO GENERATION UNIT — 611

CONVERSION UNIT — 612

← FIRST SEARCH RESULT

← SECOND SEARCH RESULT

← THIRD SEARCH RESULT

OPTIMUM SOLUTION COMPUTATION UNIT — 613

# FIG. 7

```
        ( START OF SEARCH PROCESS )
                    │
                    ▼
┌─────────────────────────────────────────┐
│      ACQUIRE COARSE-GRAINED MODEL        │──S701
└─────────────────────────────────────────┘
                    │
                    ▼
┌─────────────────────────────────────────┐
│ GENERATE TRANSFORMED COST ARITHMETIC     │
│ EXPRESSION BY OMITTING DISTINCTION       │──S702
│ BETWEEN L-FORM AND D-FORM IN COST        │
│ ARITHMETIC EXPRESSION                    │
└─────────────────────────────────────────┘
                    │
                    ▼
┌─────────────────────────────────────────┐
│  INSTRUCT TO SEARCH FOR OPTIMUM          │
│  ARRANGEMENT COMBINATION, USING          │
│  TRANSFORMED COST ARITHMETIC             │──S703
│  EXPRESSION AND CONSTRAINT TERM          │
│  ARITHMETIC EXPRESSION                   │
└─────────────────────────────────────────┘
                    │
                    ▼
┌─────────────────────────────────────────┐
│      ACQUIRE FIRST SEARCH RESULT         │──S704
└─────────────────────────────────────────┘
                    │
                    ▼
┌─────────────────────────────────────────┐
│  INSTRUCT TO SEARCH FOR OPTIMUM          │
│  ARRANGEMENT COMBINATION WITH FIRST      │
│  SEARCH RESULT AS INITIAL ARRANGEMENT,   │──S705
│  USING COST ARITHMETIC EXPRESSION AND    │
│  CONSTRAINT TERM ARITHMETIC EXPRESSION   │
└─────────────────────────────────────────┘
                    │
                    ▼
┌─────────────────────────────────────────┐
│     ACQUIRE SECOND SEARCH RESULT         │──S706
└─────────────────────────────────────────┘
                    │
                    ▼
┌─────────────────────────────────────────┐
│  CHANGE ANY ONE OR PLURALITY OF          │
│  COEFFICIENTS OF CONSTRAINT TERM         │
│  ARITHMETIC EXPRESSION AND GENERATE      │──S707
│  CHANGED CONSTRAINT TERM ARITHMETIC      │
│  EXPRESSION                              │
└─────────────────────────────────────────┘
                    │
                    ▼
┌─────────────────────────────────────────┐
│  INSTRUCT TO SEARCH FOR OPTIMUM          │
│  ARRANGEMENT COMBINATION WITH LATEST     │
│  SEARCH RESULT AS INITIAL ARRANGEMENT,   │──S708
│  USING COST ARITHMETIC EXPRESSION AND    │
│  CHANGED CONSTRAINT TERM ARITHMETIC      │
│  EXPRESSION                              │
└─────────────────────────────────────────┘
                    │
                    ▼
┌─────────────────────────────────────────┐
│      ACQUIRE THIRD SEARCH RESULT         │──S709
└─────────────────────────────────────────┘
                    │
                    ▼
       ╱ IS THERE ROOM ╲       S710
 YES  ╱ FOR IMPROVEMENT IN ╲
─────  THIRD SEARCH RESULT?
       ╲               ╱
        ╲             ╱
             │ NO
             ▼
┌─────────────────────────────────────────┐
│        OUTPUT OPTIMUM SOLUTION           │──S711
└─────────────────────────────────────────┘
                    │
                    ▼
        ( END OF SEARCH PROCESS )
```

## FIG. 8A

| | SEARCH RESULT | | | |
|---|---|---|---|---|
| PEPTIDE | NUMBER OF RESIDUES | NUMBER OF BITS | COST | TIME |
| A | 12 | 60,671 | -1282 | 255 |
| B | 10 | 42,918 | -966 | 188 |
| C | 15 | 167,256 | -926 | 855 |

## FIG. 8B

| PEPTIDE | NUMBER OF RESIDUES | FIRST STAGE | | | SECOND STAGE | | | THIRD STAGE | | | AGGREGATE TIME |
|---|---|---|---|---|---|---|---|---|---|---|---|
| | | NUMBER OF BITS | COST | TIME | NUMBER OF BITS | COST | TIME | COEFFICIENT | COST | TIME | |
| A | 12 | 8,192 | -1387 | 41 | 60,671 | -1357 | 0 | ×0.6 | -1357 | 0 | 41 |
| B | 10 | 8,192 | -960 | 42 | 42,918 | -930 | 0 | ×0.6 | -1001 | 0 | 42 |
| C | 15 | 14,298 | -1172 | 712 | 167,256 | -1112 | 1 | ×0.8 | -1132 | 1 | 714 |

Europäisches
Patentamt

European
Patent Office

Office européen
des brevets

## EUROPEAN SEARCH REPORT

**Application Number**

EP 22 17 6981

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WASSENAAR TSJERK A. ET AL: "Going Backward: A Flexible Geometric Approach to Reverse Transformation from Coarse Grained to Atomistic Models", JOURNAL OF CHEMICAL THEORY AND COMPUTATION: JCTC, vol. 10, no. 2, 11 February 2014 (2014-02-11), pages 676-690, XP055980509, US ISSN: 1549-9618, DOI: 10.1021/ct400617g * p. 678, 680-682, 684, fig. 5 * | 1-10 | INV. G16B15/20 |
| A | LOISON CLAIRE ET AL: "Multi-scale modeling of mycosubtilin lipopeptides at the air/water interface: structure and optical second harmonic generation", PHYSICAL CHEMISTRY CHEMICAL PHYSICS, vol. 16, no. 5, 1 January 2014 (2014-01-01), pages 2136-2148, XP055980514, ISSN: 1463-9076, DOI: 10.1039/C3CP53101E * the whole document * | 1-10 | |
| | | | **TECHNICAL FIELDS SEARCHED (IPC)** |
| | | | G16B |

The present search report has been drawn up for all claims

| Place of search | Date of completion of the search | Examiner |
|---|---|---|
| Munich | 11 November 2022 | Lüdemann, Susanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                      

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019159683 A
- JP 2015007537 A
- JP 2021082165 A